# EUROPEAN PATENT APPLICATION

(11) **EP 4 616 885 A1**
(43) Date of publication of application: **17.09.2025**
(21) Application number: 24205599.4
(22) Date of filing: 09.10.2024
(51) Int. Cl.: A61M 5/24

(54) **RELEASE MECHANISM FOR A DRUG DELIVERY DEVICE**

(71) Applicant: Ypsomed AG, 3401 Burgdorf (CH)
(72) Inventor: Loser, Peter, 3037 Herrenschwanden (CH); Bernhard, Mario, 3400 Burgdorf (CH); Wegner, Stephan, 4051 Basel (CH); Shepherd, David, GB-EH11 2LX Edinburgh (GB); Fischbacher, Peter Alastair, GB-EH11 2LX Edinburgh (GB); McLusky, James Donald, EH6 4QN Edinburgh (GB)

(57) **Abstract**

The present invention relates to a release mechanism for a drug delivery device for dispensing a liquid drug from a reservoir through a needle, the release mechanism comprising a drive unit and a reservoir unit adapted to hold the reservoir and releasably connectable to the drive unit. The drive unit comprises a drive unit housing defining a longitudinal axis, a plunger movable inside the drive unit housing in a dispensing direction to dispense the liquid drug and a mechanical drive, preferably a compression spring, arranged inside the drive unit housing for moving, when loaded, the plunger in the dispensing direction. The reservoir unit comprises a reservoir unit housing and a needle cover movable inside the reservoir unit housing between an initial position, in which the needle cover covers the needle, and a retracted position, in which the needle protrudes from a distal end of the needle cover. For loading the drug delivery device, the reservoir unit is insertable into the drive unit housing from a distal end of the drive unit housing. The drive unit housing comprises a first connection element and the reservoir unit housing comprises a second connection element adapted to establish a connection between the drive unit and the reservoir unit upon contact of the first connection element and the second connection element, during insertion of the reservoir unit into the drive unit housing. According to the invention the drive unit of the release mechanism comprises a pusher sleeve movable inside the drive unit housing and adapted to move in a proximal direction when the needle cover is moved from the initial position to the retracted position, and to move in a distal direction while releasing the connection between the drive unit and the reservoir unit when the needle cover returns towards a final extended position.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from a drug delivery device comprising a preferably reusable drive unit and a preferably disposable reservoir unit configured for releasable attachment to the drive unit.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a stopper in a container barrel, wherein the drive spring may have to be charged or strained manually prior to injection of a dose. A manually powered delivery drive requires a user to manually provide the energy to move the stopper, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the stopper manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing a syringe as well as a drive mechanism in order to move the stopper of the syringe upon actuation of the autoinjector. The drive mechanism typically comprises a source of drive, such as an electric motor or a mechanical spring for moving a transfer element, for example a plunger rod, which acts on the stopper of the syringe. The prefilled syringe has a needle or cannula that is permanently attached to a first end of a syringe barrel or reservoir that is sealed at the opposing end by the stopper.

For safety and hygiene reasons it is desirable that the needle does not protrude from a housing of the autoinjector except for the time when the needle is used for injection of a medicament. Thus, either the autoinjector moves the needle out of the housing for the injection and back into the housing after injection or the autoinjector includes a needle cover which may be moved to unsheathe the needle for injection, and which may be moved back to a needle covering position after the injection.

The majority of autoinjectors are configured as single use devices which incorporate both the syringe and the drive mechanism in the same housing. Such devices are usually disposable for hygiene reasons. Disposable autoinjectors comprising an electric actuator or an electronic control require a source of energy which is usually in the form of a battery. However, in this case, the autoinjectors should not be disposed in the regular waste but have to be subjected to special disposal or to recycling, which is an additional burden to the patient. Further, disposing a battery, motor and/or electronics after a single use is a waste of resources and increases the costs of the therapy. Disposable autoinjectors comprising a mechanical drive, like a compression spring, are easier to dispose than autoinjectors with an electric drive, however it is also a waste of resources to dispose the compression spring after a single use, which also increases the costs of the therapy.

In order to account for a need to handle and dispose the autoinjector parts differently semi-reusable autoinjectors have been developed. Such autoinjectors typically comprise a reusable drive unit and a disposable syringe unit which may be releasably coupled or attached to the drive unit. The drive unit usually includes the drive mechanism and electronics whereas the syringe unit includes the syringe with the needle and a needle cover sleeve. The user can thus discard the syringe unit when it is empty or after use and can load the drive unit with a new syringe unit for a new upcoming injection. A problem for semi-reusable mechanical autoinjectors is that the compression spring has to be recompressed or re-loaded when the syringe unit is inserted. Therefore, the connection between the drive unit and the syringe unit has to be strong enough to withstand the bias of the mechanical spring but at the same time it has to be easy and safe for the user to release the connection between the syringe unit and the drive unit and to separate the syringe unit from the drive unit.

EP 0 666 084 A2, WO 18091916 A1, US 2016/354556 A1 and CN 217489449 U disclose semi-reusable autoinjectors in which the connection between the reusable drive unit and the disposable reservoir unit is released manually after the injection is completed in that the user pulls the reservoir unit out of the drive unit. A drawback of these known semi-reusable autoinjectors is that it can require a strong force to separate the reservoir unit from the drive unit, as the force for separation has to be stronger than the force exerted by the mechanical drive during the injection.

EP 2618870 A1 discloses a semi-reusable autoinjector comprising a reusable drive unit with a mechanical drive mechanism and a disposable syringe unit. To separate the syringe unit from the drive unit after the injection is completed, the user slides an eject button that rotates the syringe unit inside the drive unit so that the syringe unit can then be removed from the drive unit. A drawback of this known semi-reusable autoinjector is that the syringe unit can only be separated after the injection is completed so that there is no possibility to separate the syringe unit from the drive unit before the injection is completed, once the syringe unit is fully inserted into the drive unit.

WO 14029621 A1 discloses a semi-reusable autoinjector comprising a reusable front housing and a reusable rear housing, releasably connectable to each other, and a replaceable container assembly receivable inside the front housing and the rear housing. To remove the container assembly from the autoinjector, the user has to separate the front housing and the rear housing and then take out the container assembly. A drawback of this known semi-reusable autoinjector is that the user has to execute two steps to remove the container assembly and has no access to the container assembly as long as the front housing and the rear housing are connected.

### DESCRIPTION OF THE INVENTION

It is an objective of the present invention to develop a drug delivery device comprising a drive unit with a mechanical drive and a reservoir unit that allows a user or patient to release the reservoir unit from the drive unit in an easy and safe way after the injection is completed as well as before and during the injection.

This objective is achieved by the features of the independent claims. Preferred embodiments are evident from the dependent claims.

The invention relates to a release mechanism for a drug delivery device for dispensing a liquid drug from a reservoir through a needle.

The release mechanism comprises a drive unit and a reservoir unit adapted to hold the reservoir and releasably attachable or connectable to the drive unit. With the release mechanism, the connection between the drive unit and the reservoir unit can be released so that the reservoir unit can be separated from the drive unit. The drug delivery device is preferably an injection device and more preferably an autoinjector, that is adapted to dispense the whole content of the reservoir in a single injection stroke. Most preferably, the drug delivery device is a semi-disposable autoinjector with a reusable drive unit and a disposable reservoir unit. After each injection the reservoir unit is disposed and replaced by a new disposable reservoir unit for an upcoming new injection. The reservoir may be a prefilled syringe having a needle or cannula that is permanently attached to a first end of a syringe barrel that is sealed at the opposing end by a stopper. Alternatively, the reservoir may be a cartridge with a connecting portion at the distal end adapted to be connected to an injection needle prior injection.

The drive unit of the release mechanism comprises a drive unit housing defining a longitudinal axis and a plunger movable inside the drive unit housing in a dispensing direction to dispense the liquid drug. The plunger can also be referred to as piston rod or drive element. The longitudinal axis defines a longitudinal direction of the drive unit. The dispensing direction can preferably be the longitudinal direction and more preferably a distal direction. The drive unit housing can comprise only one part having a sleeve structure. The drive unit housing can also comprise two or more parts that together form a sleeve structure. In this alternative, the two or more drive unit housing parts can be connected mechanically, for example with screws or latches, or they can be bonded to each other, for example by gluing or welding. The drive unit further comprises a mechanical drive arranged inside the drive unit housing for moving, when loaded, the plunger in the dispensing direction. Preferably, the mechanical drive is a compression spring. For loading, the mechanical drive can preferably be compressed in a proximal direction, hence a distal end of the mechanical drive is moved in the proximal direction while the proximal end of the mechanical drive is fixed.

The reservoir unit of the release mechanism comprises a reservoir unit housing. The reservoir unit housing also defines a longitudinal axis and a longitudinal direction of the reservoir unit, that coincide with the longitudinal axis and the longitudinal direction of the drive unit, when the reservoir unit is inserted into the drive unit housing. Preferably, the reservoir unit housing is adapted to hold the reservoir. However, in other alternatives the reservoir unit can further comprise a reservoir holder arranged inside the reservoir unit housing that holds the reservoir. Preferably, after assembly of the reservoir unit, the reservoir is fixed or non-movably arranged inside the reservoir unit housing or the reservoir holder. That means that during an injection the reservoir does not move relative to the reservoir unit housing or the reservoir holder and hence also does not move relative to the drive unit housing. Therefore, the user has to insert or penetrate the injection needle into the injection site by a manual insertion force. The reservoir unit further comprises a needle cover or cover member movable inside the reservoir unit housing along the longitudinal axis of the reservoir unit housing between an initial position, in which the needle cover covers the needle, and a retracted position, in which the needle is uncovered and protrudes from a distal end of the needle cover. The needle cover is guided by the reservoir unit housing. Preferably, the needle cover can be movable inside the reservoir unit housing, with other words be enclosed by the reservoir unit housing. In alternative embodiments, the needle cover can also enclose the reservoir unit housing, hence be arranged around the reservoir unit housing. The needle cover ensures that the needle is covered before and after the injection, so that the patient cannot accidentally touch the needle. The needle cover may be implemented as shield or sleeve or sleeve-shaped element.

For loading the drug delivery device, the reservoir unit is insertable into the drive unit housing from a distal end of the drive unit. Preferably the drive unit is inserted by a user or patient prior to an injection to assemble the drug delivery device and to prepare the drug delivery device for the injection. This means the drug delivery device is not fully assembled by the manufacturer before the reservoir unit and the drive unit are delivered to the user as separate parts.

The drive unit housing comprises a first connection element and the reservoir unit housing comprises a second connection element that are adapted to establish a connection between the drive unit and the reservoir unit upon contact or engagement of the first connection element and the second connection element. The contact between the first connection element and the second connection element occurs during insertion of the reservoir unit into the drive unit housing. Preferably the first connection element and the second connection element connect or attach the reservoir unit to the drive unit in a predefined end position of the reservoir unit. This means that by inserting the reservoir unit into the drive unit housing and thereby establishing the connection between the reservoir unit and the drive unit, the user or patient creates a functional drug delivery device that is ready to use for an injection. The first connection element and the second connection element have to be adapted to be able to provide the connection between the reservoir unit and the drive unit against the force of the mechanical drive and potentially against the force of removing a protective cap from the reservoir unit after attaching the reservoir unit to the drive unit and before the injection. With other words, the connection between the reservoir unit and the drive unit has to be strong enough to hold the reservoir unit inside the drive unit housing against the force of the mechanical drive and potentially the force of removing the protective cap. Preferably, the first connection element and/or the second connection element is elastically deformable, preferably by the other connection element, to establish the connection. This means that during the insertion of the reservoir unit into the drive unit housing, the first connection element and/or the second connection element is elastically deformed to be able to pass the other connection element and then returns to its undeformed state in which it is not able to pass the other connection element, which establishes the connection.

According to the invention, the drive unit of the release mechanism comprises a pusher sleeve movable inside the drive unit housing and adapted to move in a proximal direction when the needle cover is moved from the initial position to the retracted position, and to move in a distal direction while releasing the connection between the drive unit and the reservoir unit, established by the connection elements, when the needle cover returns towards a final extended position. In the context of the present invention, releasing the connection means a spontaneous separation of the connection, so that the user can separate the reservoir unit and the drive unit by moving the reservoir unit in the distal direction with respect to the drive unit, with other words by pulling the reservoir unit out of the drive unit. This means the connection between the reservoir unit and the drive unit is automatically released when the needle cover returns or moves towards the final extended position.

The user can then easily pull the reservoir unit out of the drive unit only requiring a small force because the connection is already released by the pusher sleeve. Preferably, the needle cover moves in the proximal direction when the drug delivery device is pressed against the skin of the patient at a desired injection site, which pushes the needle cover into the drive unit housing, hence causes a proximal movement of the needle cover relative to the reservoir unit housing and the drive unit housing. This can preferably also release the plunger to start the injection. When the needle cover is pushed in the proximal direction, the pusher sleeve is also pushed in the proximal direction relative to the drive unit housing by the needle cover. When the user removes the drug delivery device from the skin, the needle cover moves back or returns in the distal direction relative to the reservoir unit housing and the drive unit housing to the final extended position. This also allows the pusher sleeve to move in the distal direction relative to the drive unit housing while automatically releasing the connection between the reservoir unit and the drive unit. Normally, the user removes the drug delivery device from the skin after the injection is completed, but it is also possible to remove the drug delivery device earlier before the injection is completed, hence during the injection. Preferably, the needle cover and the pusher sleeve can be biased in the distal direction by a pusher sleeve spring that is arranged between the drive unit housing and the pusher sleeve and hence also acts on the needle cover via the pusher sleeve. Preferably, the pusher sleeve elastically deforms the first connection element and/or the second connection element so that the first connection element and the second element can pass by each other, which releases the connection.

An advantage of the release mechanism according to the invention is that the connection between the drive unit and the reservoir unit is released automatically when the needle cover returns from the retracted position to the final extended position. Hence, for the user no additional step is necessary to release the connection between the drive unit and the reservoir unit. Another advantage of the inventive release mechanism is that it is easier for the user to separate the reservoir unit from the drive unit, because the connection is already released so that the user does not need to overcome a high holding force to remove the reservoir unit from the drive unit. This enables the user to remove the reservoir unit from the drive unit in a safe and easy way and at the same time the connection between the reservoir unit and the drive unit can be adapted to provide a strong holding force before the connection is released.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The term "distal" is meant to refer to the direction or the end of the drug delivery device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion device" refers to a device with a cannula or needle that remains in the skin of the patient for a prolonged period of time, for example, several hours.

In the claims, the word "comprising" or "comprises" does not exclude other elements or steps, and the indefinite article "a" or "an" does not exclude a plurality. For example, "an arm" does not exclude the fact that there may be two arms that functionally or structurally fulfill the purpose of "an arm". The mere fact that certain elements or steps are recited in distinct claims shall not preclude the existence of further meaningful combinations of these elements or steps.

In a preferred embodiment, the reservoir unit is insertable into the drive unit housing by a linear movement along the longitudinal axis, hence in the longitudinal direction. Additionally or alternatively, the reservoir unit is separable from the drive unit also by a linear movement along the longitudinal axis, hence in the longitudinal direction, in the opposite direction as for the insertion. This allows for a simple design of the drug delivery device and provides an easy and comfortable inserting of the reservoir unit into the drive unit and separating of the reservoir unit from the drive unit.

In another preferred embodiment, the pusher sleeve comprises a release arm adapted to elastically deform the first connection element or the second connection element to release the connection between the reservoir unit and the drive unit. Preferably, the release arm deforms the first connection element or the second connection element in the radial direction. To deform the respective connection element, the connection arm contacts the connection element, while the pusher sleeve moves in the distal direction. This allows for a simple design of the pusher sleeve. Preferably, the drive unit housing comprises two first connection elements arranged opposite each other in a circumferential direction and the reservoir unit housing comprises two second connection elements arranged opposite each other in the circumferential direction. For this alternative, the pusher sleeve comprises two release arms also arranged opposite each other in the circumferential direction and adapted to release the connection between both pairs of connection elements at the same time.

Preferably, the pusher sleeve further comprises a contact arm with a distal end surface adapted to contact a proximal end surface of the needle cover, after the reservoir unit is inserted into the drive unit housing. Hence, the release arm respectively the two release arms are not needed to push the pusher sleeve in the proximal direction and can specifically be designed to release the connection between the reservoir unit and the drive unit. Preferably, the pusher sleeve comprises two release arms and two contact arms and the contact arms are located radially inside the release arms and/or offset to the release arms in a circumferential direction. The two contact arms enable a straight movement of the pusher sleeve without tilting of the pusher sleeve and the two contact arms enable a simultaneous release of two first connection elements and two second elements as described above.

In a preferred embodiment, the drug delivery device further comprises a pusher sleeve spring adapted to urge the pusher sleeve and the needle cover in the distal direction. Hence, the pusher spring moves the pusher sleeve and the needle cover in the distal direction as soon as the needle cover is removed from the skin of the patient. The pusher sleeve spring is arranged between a proximal end surface of the pusher sleeve and a proximal inner end of the drive unit housing. This allows for an easy implementation of the pusher sleeve movement into the release mechanism and the drug delivery device.

In a preferred embodiment, the needle cover comprises a needle cover clip elastically deformable in the radial direction and the reservoir unit housing comprises a recess and a channel. The recess is adapted to receive the needle cover clip in the initial position of the needle cover and the channel is adapted to receive the needle cover clip in the retracted position of the needle cover and to guide the needle cover to the final extended position. The final extended position is distal from the initial position, hence, in the final extended position, the needle cover protrudes further from a distal end of the reservoir unit housing than in the initial position. This means that in the initial state, the needle cover protrudes form the distal end of the reservoir unit housing to cover the needle and the needle cover clip is received in the recess of the reservoir unit housing. When the needle cover is pushed against the skin at the injection site, the needle cover clip is elastically deformed in the radial direction so that the needle cover can move in the proximal direction until the needle cover clip reaches the proximal end of the channel in the reservoir unit housing in the retracted position of the needle cover. In the retracted position the needle cover clip is received in the channel so that the needle cover clip returns to the undeformed state of the needle cover clip. When the needle cover is removed from the injection site, the needle cover moves in the distal direction to the final extended position, wherein the needle cover clip is guided to the distal end of the channel. This allows for an easy implementation of the needle cover movement into the release mechanism and the drug delivery device.

Preferably, the needle cover comprises a lockout clip positioned closer to the distal end of the needle cover than the needle cover clip and adapted to lock the needle cover in the final extended position by abutting the distal end surface of the reservoir unit housing. The lockout clip is elastically deformable and guided within the reservoir unit housing until the needle cover reaches its final extended position. In the final extended position, the lockout clip returns to an undeformed state and abuts the distal end surface of the reservoir unit housing. This makes sure that the needle cover cannot be moved in the proximal direction, so that the needle cannot be uncovered after the needle cover has reached its final extended position. In alternative embodiments, the lockout clip can abut a locking protrusion on an inner surface of the reservoir unit housing.

Preferably, the pusher sleeve is adapted to release the connection between the drive unit and the reservoir unit at the same time or after the needle cover is locked in the final extended position. With other words the connection is not released before the needle cover is locked in the final extended position. This ensures that the needle cover is locked when the user separates the reservoir unit from the drive unit so that the needle cover cannot move in the proximal direction and reveal the needle. This ensures that the user does not accidentally touch the needle while pulling the reservoir unit out of the drive unit. This can be achieved by adapting the geometry of the pusher sleeve and the channel in the reservoir unit housing, in particular regarding their length and position in the longitudinal direction, so that the needle cover reaches the final extended position before the pusher sleeve respectively the release arm reaches the first connection element and the second connection element.

In a preferred embodiment, the first connection element comprises a projection on an inner surface of the drive unit housing and the second connection element comprises a projection on an outer surface of the reservoir unit housing, preferably at a proximal end of the reservoir unit housing. The projections are easy to manufacture so that this embodiment provides an easy and cheap way to establish the connection between the reservoir unit and the drive unit.

Preferably, the first connection element comprises a proximal abutment surface facing in the proximal direction and the second connection element comprises a distal abutment surface facing in the distal direction. After the reservoir unit is inserted into the drive unit housing, the first connection element is positioned distal of the second connection element, so that the abutment surfaces contact each other. This means that the first connection element and/or the second connection element is elastically deformed while the second connection element passes by the first connection element and then returns to its undeformed position when it has passed the other connection element so that the abutment surfaces abut each other and hold the reservoir unit inside the drive unit housing.

In a preferred embodiment, the second connection element is arranged on a free end of an elastically deformable reservoir clip at a proximal end of the reservoir unit housing. In particular the free end of the reservoir clip is the distal end of the reservoir clip. This configuration provides a second connection element with a high holding force that at the same time can be elastically deformed in the radial direction in an easy way to enable the second connection element to pass the first connection element.

Preferably, the reservoir clip is adapted to be deformed in the radial direction by the first connection element. Therefore, no further features are necessary to deform the reservoir clip in the radial direction to enable the inserting of the reservoir unit into the drive unit housing, which allows a simple design of the drug delivery device.

In a preferred embodiment, the connection between the drive unit and the reservoir unit is alternatively releasable by an emergency eject button, wherein the second connection element is deformable by the emergency eject button. With other words, instead of being deformed by the pusher sleeve when the pusher sleeve moves in the distal direction, the second connection element can alternatively be deformed by the emergency eject button to release the connection between the reservoir unit and the drive unit. This is for example necessary when the user has inserted a wrong reservoir unit, meaning the reservoir contains a wrong or expired medicament, or when the injection fails to complete. In this case the user can release the connection immediately at any time by pressing the emergency eject button.

In a preferred embodiment, the drive unit housing comprises a bracket on an inner surface of the drive unit housing for guiding the pusher sleeve. This provides a simple and easy way to guide the pusher sleeve in the drive unit housing to ensure a linear movement of the pusher sleeve without rotation or tilting of the pusher sleeve, so that the connection between the reservoir unit and the drive unit is reliably released when the pusher sleeve moves in the distal direction.

The invention also relates to a drug delivery device comprising an inventive release mechanism as described above. Preferably, the drive unit housing, the reservoir unit housing, the plunger, the needle cover and/or the pusher sleeve are injection-molded thermoplastic plastic parts. The injection-molded parts allow for a cost-effective production of the reservoir unit parts, in particular in case of a high-volume production. Alternatively or additionally, the drug delivery device is a semi-reusable autoinjector with a reusable drive unit and a disposable reservoir unit.

### BRIEF DESCRIPTION OF THE DRAWINGS

The subject matter of the invention will be explained in more detail in the following text with reference to preferred example embodiments which are illustrated in the attached drawings, in which depict:
- Fig. 1: an example embodiment of an inventive drug delivery device before the reservoir unit is inserted into the drive unit;
- Fig. 2: the drug delivery device of fig. 1 after the reservoir unit is inserted into the drive unit;
- Fig. 3: an exploded view of the drug delivery device of fig. 1 and 2;
- Fig. 4: an exploded view of the reservoir unit of the drug delivery device of fig. 1 to 3;
- Fig. 5: a sectional view of the drug delivery device of fig. 1 to 3 while the reservoir unit is inserted into the drive unit;
- Fig. 6: a side view of the drug delivery device of fig. 1 to 5 after the reservoir unit is inserted into the drive unit with the needle cover in the initial position;
- Fig. 7: a sectional view of the drug delivery device of fig. 6 along line VII - VII in fig. 6;
- Fig. 8: another side view of the drug delivery device of fig. 1 to 7 after the reservoir unit is inserted into the drive unit with the needle cover in the initial position;
- Fig. 9: a sectional view of the drug delivery device of fig. 8 along line IX - IX in fig. 8;
- Fig. 10: a perspective view of the reservoir unit housing of the drug delivery device of fig. 1 to 9;
- Fig. 11: the drug delivery device of fig. 1 to 10 with the needle cover in the retracted position;
- Fig. 12: detail XII of fig. 11 in an enlarged view;
- Fig. 13: a side view of the drug delivery device of fig. 1 to 12 with the needle cover in the final extended position;
- Fig. 14: a sectional view of the drug delivery device of fig. 13 along line XIV - XIV in fig. 13;
- Fig. 15: another side view of the drug delivery device of fig. 1 to 14 with the needle cover in the final extended position;
- Fig. 16: a sectional view of the drug delivery device of fig. 15 along line XVI - XVI in fig. 15;
- Fig. 17: a sectional view of the drive unit of the drug delivery device of fig. 1 to 16;
- Fig. 18: a perspective view of the needle cover of the drug delivery device of fig. 1 to 16;
- Fig. 19: a perspective view of the reservoir unit of the drug delivery device of fig. 1 to 16 with the needle cover in the initial position;
- Fig. 20: a sectional view of the reservoir unit of fig. 19 with the needle cover in the initial position;
- Fig. 21: a sectional view of the reservoir unit of fig. 19 and 20 with the needle cover in the retracted position;
- Fig. 22: a sectional view of the drug delivery device of fig. 1 to 16 after the connection between the drive unit and the reservoir unit is released;
- Fig. 23: detail XXIII of fig. 22 in an enlarged view;
- Fig. 24: a sectional view of the drug delivery device of fig. 1 to 16 while the reservoir unit is separated from the drive unit; and
- Fig. 25: detail XXV of fig. 24 in an enlarged view.

The reference symbols used in the drawings, and their primary meanings, are listed in summary form in the list of designations. In principle, identical parts are provided with the same reference symbols in the figures.

### DETAILED DESCRIPTION OF THE PREFFERRED EMBODIMENTS

In the present description the term "distal" refers to the side where the injection needle is located. This is on the right side or the lower part of the figures. The term "proximal" refers to the opposite side or rear end of the device. This is on the left side or the upper part of the figures.

The figures show an example embodiment of an inventive drug delivery device 1 for dispensing a liquid drug from a reservoir through a needle in form of a semi-reusable autoinjector.

The semi-reusable autoinjector 1 comprises a reusable drive unit 3 and a disposable reservoir unit 5 releasably attachable or connectable to the drive unit 3. The autoinjector 1 further comprises an inventive release mechanism to release the reservoir unit 5 from the drive unit 5 to be able to separate the reservoir unit 5 from the drive unit 3.

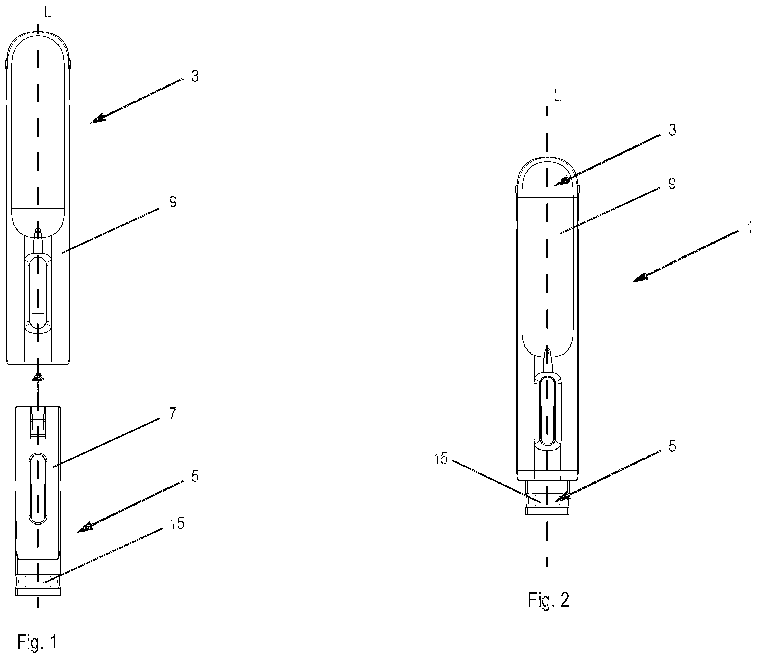

The reservoir unit 5 is insertable into the drive unit 3 from a distal end of the drive unit 3 by a linear movement in a longitudinal direction along a longitudinal axis L defined by the drive unit 3, as indicated by the arrow in fig. 1. The insertion of the reservoir unit 5 into the drive unit 3 is visible from a comparison of fig. 1 and fig. 2. Fig. 1 shows the autoinjector before the reservoir unit 5 is inserted into the drive unit 3 and fig. 2 shows the fully assembled autoinjector 1 after the reservoir unit 5 is inserted into the drive unit 3. The drive unit 3 of the example embodiment has a length of 160 mm in the longitudinal direction and an elliptical cross section with outer dimensions of 30.4 mm and 24.8 mm. The reservoir unit 5 of the example embodiment has a length of 90.1 mm in the longitudinal direction and a rectangular cross section with rounded edges and outer dimensions of 21.6 mm and 19.2 mm. The fully assembled autoinjector 1 as shown in fig. 2 has a total length of 175.6 mm, hence a major part of the reservoir unit 5 is inserted into the drive unit 3. This allows for a compact design of the autoinjector 1.

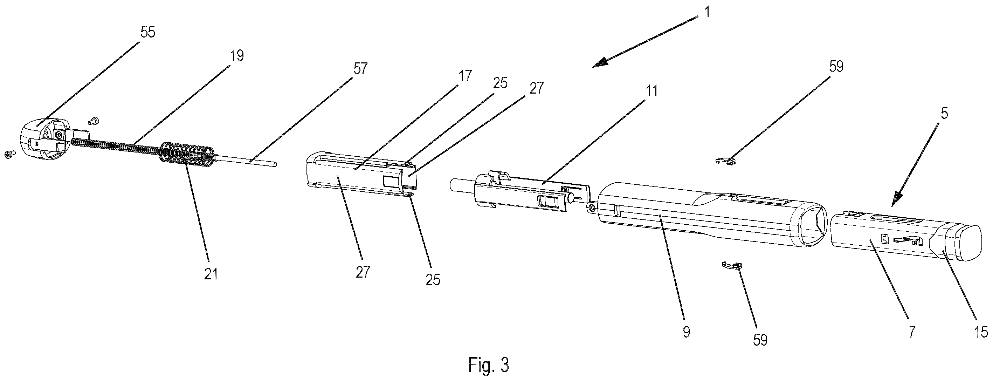

Fig. 3 shows the autoinjector 1 in an exploded view that gives an overview of the separate components of the drive unit 3 of the autoinjector 1. The main components of the drive unit 3 are a drive unit housing 9 that defines the longitudinal axis L, a plunger 11 movable inside the drive unit housing 9 in a dispensing direction to dispense the liquid drug, a pusher sleeve 17, a mechanical drive 19 in form of a compression spring to move, when loaded, the plunger 11 in the dispensing direction, and a pusher sleeve spring 21, also in form of a compression spring. In the example embodiment, the drive unit housing 9 comprises a separate drive unit cap 55 that closes the drive unit housing 9 at a proximal end. The drive unit cap 55 is connectable to the drive unit housing 9 by elastically deformable clips on the drive unit cap 55 and backed up by a screw and nut connection and corresponding screw holes in the drive unit housing 9 and the drive unit cap 55. In other embodiments, the drive unit cap 55 can also be formed integral with the rest of the drive unit housing 9. The drive unit 3 further comprises a mechanical drive rod 57 that is arranged inside the mechanical drive 19. The mechanical drive rod 57 prevents the mechanical drive 19 from bending during compression and relaxation of the mechanical drive 19.

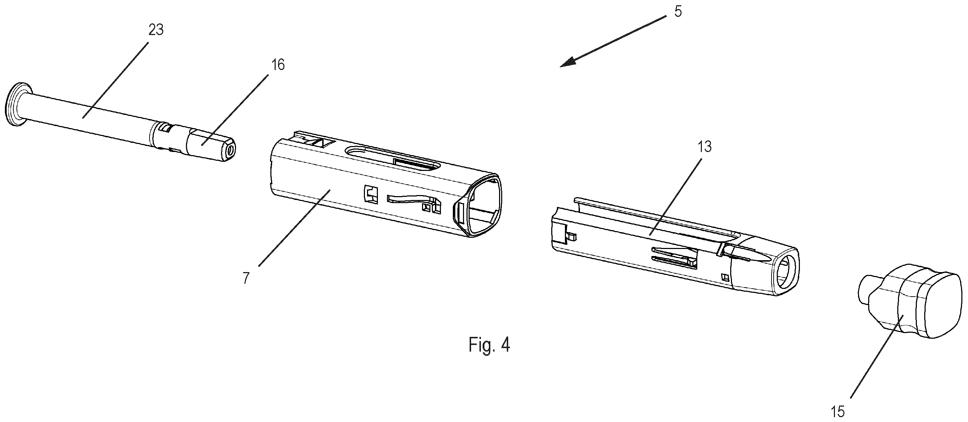

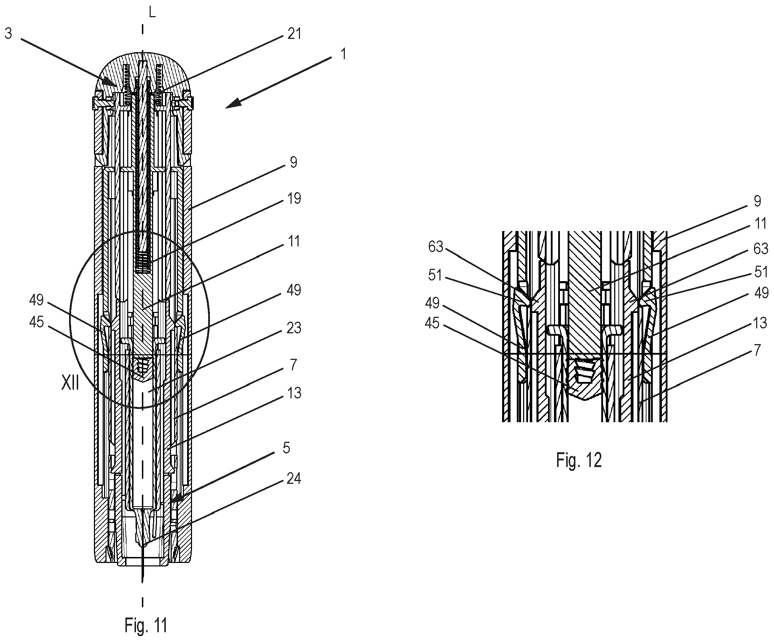

Fig. 4 shows the reservoir unit 5 of the autoinjector 1 in an exploded view that gives an overview of the separate components of the reservoir unit 5. The reservoir unit 5 comprises a reservoir unit housing 7, a needle cover 13, a cap 15 and a reservoir 23 with a needle. The reservoir 23 is a prefilled syringe with a stopper at a proximal end and the needle permanently attached to a distal end of the reservoir 23. The reservoir 23 further comprises a needle shield 16 that covers and protects the needle and has to be removed before the injection. The needle cover 13 is movable inside the reservoir unit housing 7 between an initial position, in which the needle cover 13 covers the needle, and a retracted position, in which the needle protrudes from a distal end of the needle cover 13. The movement of the needle cover 13 will be explained in detail further below with reference to fig. 11 to 21.

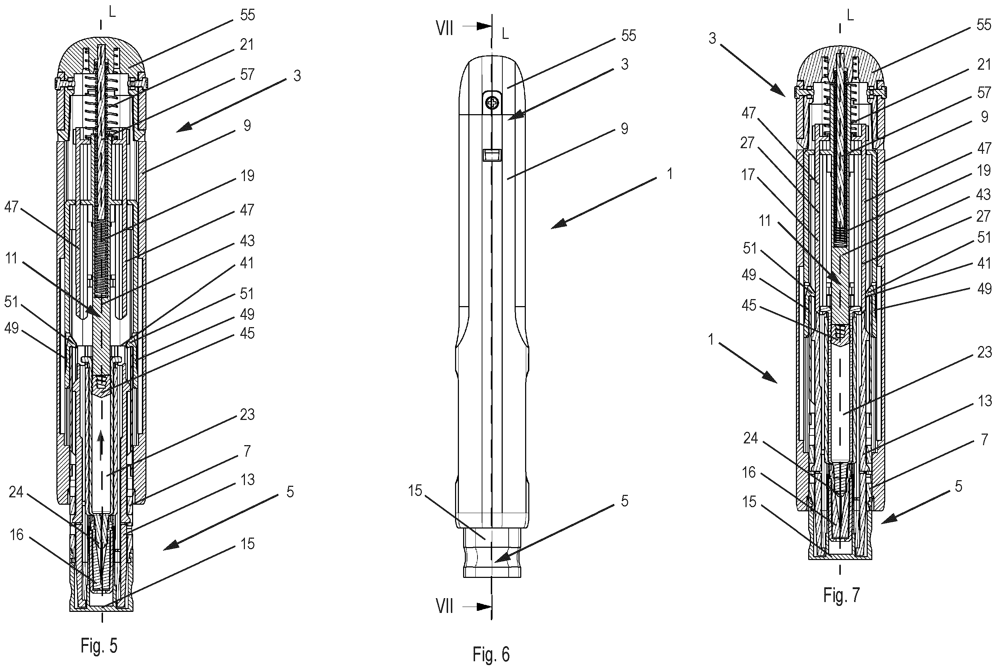

Fig. 5 shows the drive unit 3 and the reservoir unit 5 in a cross-sectional view while the reservoir unit 5 is inserted into the drive unit housing 9, as indicated by the arrow in fig. 5. While the reservoir unit 5 is inserted into the drive unit housing 9, a proximal end surface of the reservoir unit housing 7 contacts the plunger 11 and pushes the plunger 11 in the proximal direction to load the mechanical drive 19 by compressing the compression spring. The plunger 11 comprises an inner rod section 43 with a distal end surface adapted to contact and move the stopper 45 of the reservoir 23 in the distal direction for dispensing the liquid drug and two plunger arms 47 arranged radially offset from the inner rod section 43 adapted to contact the reservoir unit housing 7, so that the plunger 11 is pushed in the proximal direction when the reservoir unit 5 is inserted into the drive unit housing 9. The inner rod section 43 has a sleeve-shaped proximal section that defines an interior adapted to receive the mechanical drive 19 therein and forms a proximal end surface adapted to serve as contact surface for the distal end of the mechanical drive 19. Each plunger arm 47 comprises an elastically deformable plunger clip 49 with a projection 51 on a free end of the plunger clip 49. When the reservoir unit 5 is inserted into the drive unit housing 9, the reservoir unit housing 7 contacts the projections 51 on the plunger clips 49 to move the plunger 11 in the proximal direction. The plunger clips 49 further serve to lock the plunger 11 before the user intends to start the injection and to release the plunger 11 in order to start the injection, which will be explained in detail further below with regard to fig. 11 and 12. In the example embodiment, the plunger clips 49 are located on a distal end of the plunger arms 47 and the projections 51 are located on a free proximal end of the plunger clips 49. The distal end of each plunger clip 49 connects the plunger clip 49 to the respective plunger arm 47.

Fig. 6 to 9 show the fully assembled autoinjector 1 when the reservoir unit 5 has reached a predefined end position within the drive unit housing 9. As can be seen from a comparison of fig. 5 and 7, by inserting the reservoir unit 5 into the drive unit housing 9, the reservoir unit 5 is moved to the end position within the drive unit housing 9 and thereby pushes the plunger 11 in the proximal direction. The end position corresponds to a loaded state of the mechanical drive 19 due to a compression of the mechanical drive 19 between a proximal inner end of the drive unit cap 55 as part of the drive unit housing 9 and the proximal end surface of the inner rod section 43 of the plunger 11. In the end position, the distal end surface of the reservoir unit housing 7 is on level with the distal end surface of the drive unit housing 9.

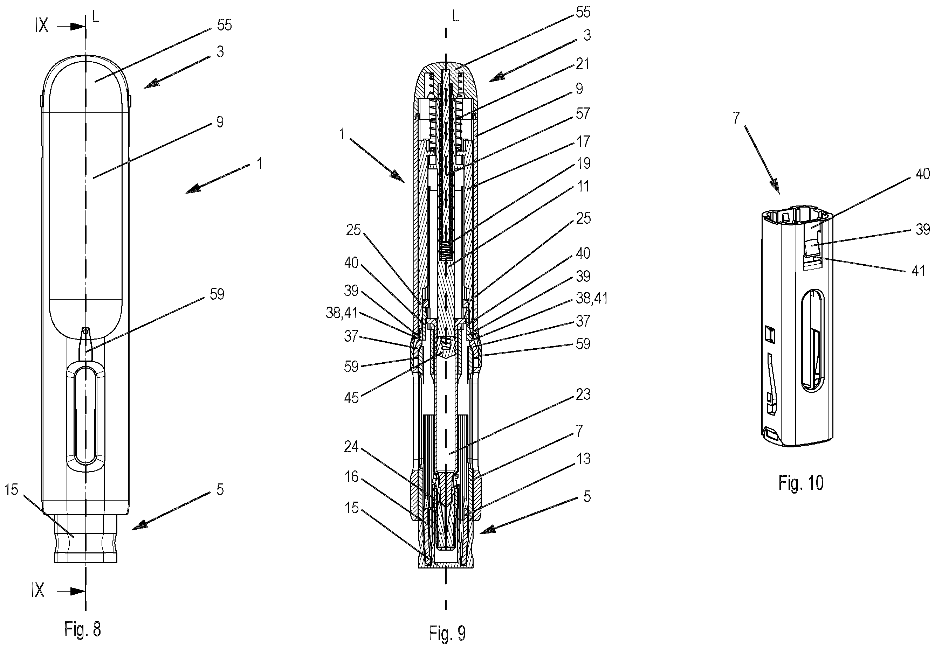

As shown in fig. 9, in the end position of the reservoir unit 5, the reservoir unit 5 is held in place against the force of the loaded mechanical drive 19 by a connection established by a first connection element 37 of the drive unit housing 9 and a second connection element 39 of the reservoir unit housing 7 as long as the connection elements 37, 39 are in contact with each other. The contact between the first connection element 37 and the second connection element 39 is established when the reservoir unit 5 is inserted into the drive unit housing 9. The first connection element 37 is formed by two projections on an inner surface of the drive unit housing 9 opposite to each other. The second connection element 39 is formed by two projections each on an outer surface of a free end of an elastically deformable reservoir clip 40. The two reservoir clips 40 are arranged opposite to each other near the proximal end of the reservoir unit housing 7 and elastically deformable in the radial direction. In the example embodiment, the free end of the reservoir clips 40 is the distal end of the reservoir clips 40, meaning that the reservoir clips 40 are connected to the rest of the reservoir unit housing 7 at a proximal end of the reservoir clips 40. Fig. 10 shows the reservoir unit housing 7 with the elastically deformable reservoir clips 40 and the projections 39 in detail.

During the insertion, the projections 39 on the reservoir clips 40 first approach the projections 37 of the drive unit housing 9 and then pass the projections 37 of the drive unit housing 9. While passing the projections 37 of the drive unit housing 9, the reservoir clips 40 are deformed in the radial direction by the projections 37. After the projections 39 of the reservoir unit housing 7 have passed the projections 37 of the drive unit housing 9, the reservoir clips 40 return to their undeformed state. The projections 37 of the drive unit housing 9 each comprise a proximal abutment surface 38 facing in the proximal direction and the protrusions 39 of the reservoir unit housing 7 each comprise a distal abutment surface 41 facing in the distal direction. When the projections 39 of the reservoir unit housing 7 have passed the projections 37 of the drive unit housing 9, the projections 37 are positioned distal of the projections 39. As the reservoir clips 40 are then back in their undeformed state, the abutment surfaces 38, 41 contact each other, so that the reservoir unit 5 cannot move in the distal direction out of the drive unit housing 9. This provides the connection between the reservoir unit 5 and the drive unit 3. When the reservoir unit 5 and the drive unit 3 are connected by the connection elements 37, 39, the plunger 11 is held by means of the projections 51 abutting the reservoir unit housing 7, so that the plunger 11 cannot move in the distal direction to dispense the medicament from the reservoir 23.

As can be seen in fig. 7, in the end position of the reservoir unit 5 within the drive unit housing 9, a proximal end surface of needle cover 13 engages with a distal end surface of the pusher sleeve 17. The pusher sleeve 17 is movable inside the drive unit housing 9 along the longitudinal axis L, so that a movement of the needle cover 13 is transferred to a movement of the pusher sleeve 17 after the insertion of the reservoir unit 5 into the drive unit housing 9. This means that the pusher sleeve 17 moves in the proximal direction, when the needle cover 13 moves in the proximal direction, and moves in the distal direction, when the needle cover 13 moves in the distal direction.

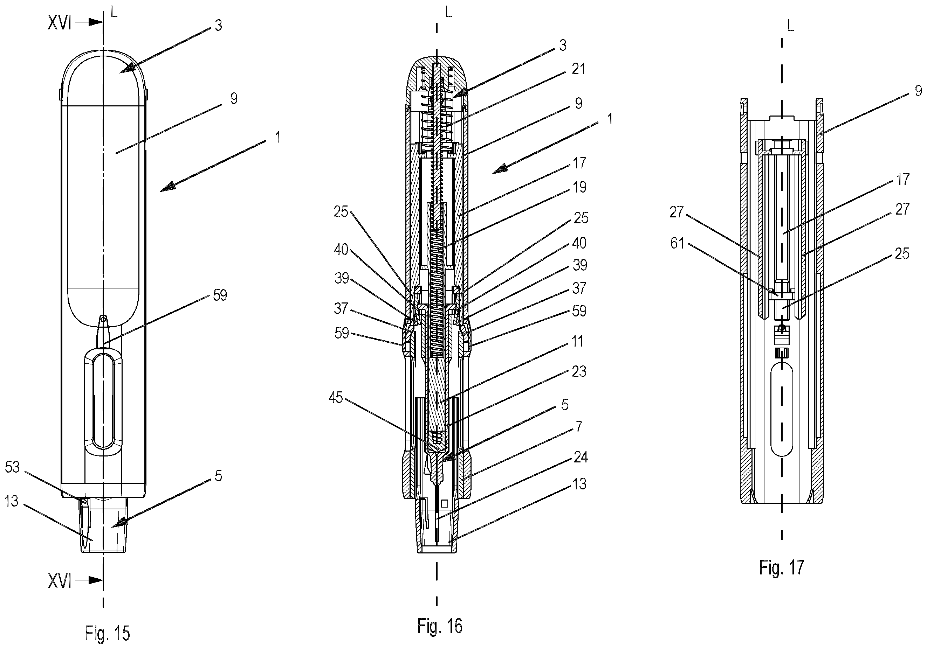

The pusher sleeve 17 comprises two release arms 25 shown in fig. 9 and 16, adapted to elastically deform the reservoir clips 40 in the radial direction to release the connection between the reservoir unit 5 and the drive unit 3 established by the connection elements 37, 39. The pusher sleeve 17 further comprises two contact arms 27 shown in fig. 7, each with a distal surface adapted to contact the proximal surface of the needle cover 13 to transmit the movement of the needle cover 13 to a movement of the pusher sleeve 17. As shown in fig. 3, 7 and 9, the contact arms 27 are located radially inside the release arms 25 and offset to the release arms 25 in a circumferential direction.

Before the user can use the autoinjector 1 to make an injection, the user has to remove the cap 15 from the autoinjector 1 by manually pulling the cap 15 in the distal direction. In other embodiments the cap can be removed by turning the cap. The cap 15 is engaged with the needle shield 16 of the reservoir 23, so that when the cap 15 is removed, the needle shield 16 is removed together with the cap 15. When the cap is removed, the needle 24 is still covered by the needle cover 13, so that the user cannot accidentally touch the needle 24. In this initial position, the needle cover 13 protrudes from the distal end of the reservoir unit housing 7 and surrounds the needle 24.

To start the injection, the user presses the autoinjector 1 against the skin at a desired injection site. This causes the needle cover 13 to move from the initial position in the proximal direction with respect to the reservoir unit housing 7 and the reservoir 23 to a retracted position as shown in fig. 11.

In the retracted position, the needle 24 protrudes from a distal end of the needle cover 13 so that it can penetrate the skin. While the needle cover 13 moves in the proximal direction, the needle cover 13 elastically deforms the plunger clips 49 in the radial direction from an initial position of the plunger clip 49 in which the plunger clips 49 hold the plunger 11 against the bias of the loaded mechanical drive 19 to a deformed position of the plunger clips 49. Therefore, the needle cover 13 comprises protrusions 63 with ramped proximal surfaces on an outer surface of the needle cover 13. As particularly shown in fig. 12, when the plunger clips 49 are deformed, the projections 51 on the plunger clips 49 no longer abut the reservoir unit housing 7, so that the plunger 11 is released and is free to move in the distal direction under the bias of the loaded mechanical drive 19 to start the injection and to expel the medicament from the reservoir 23. The trigger point for the release of the plunger 11 is shortly before the needle cover 13 reaches its retracted position. In fig. 11 the injection process has not started. Therefore, the full amount of medicament is still in the reservoir 23 and the stopper 45 of the reservoir 23 is in its most proximal position. As can be seen in fig. 11, the pusher sleeve 17 moves together with the needle cover 13 in the proximal direction. Hence, the movement of the needle cover 13 in the proximal direction also causes the pusher sleeve 17 to move in the proximal direction.

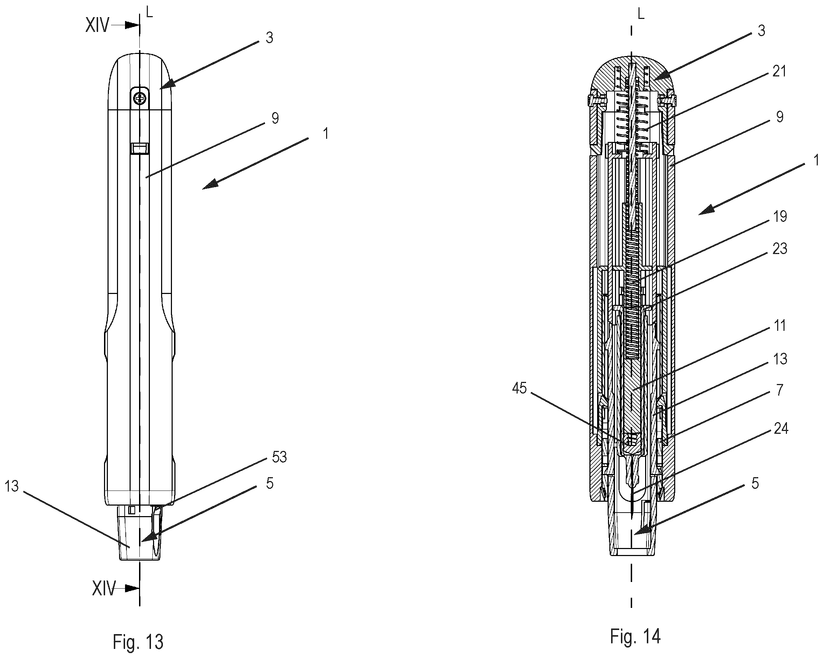

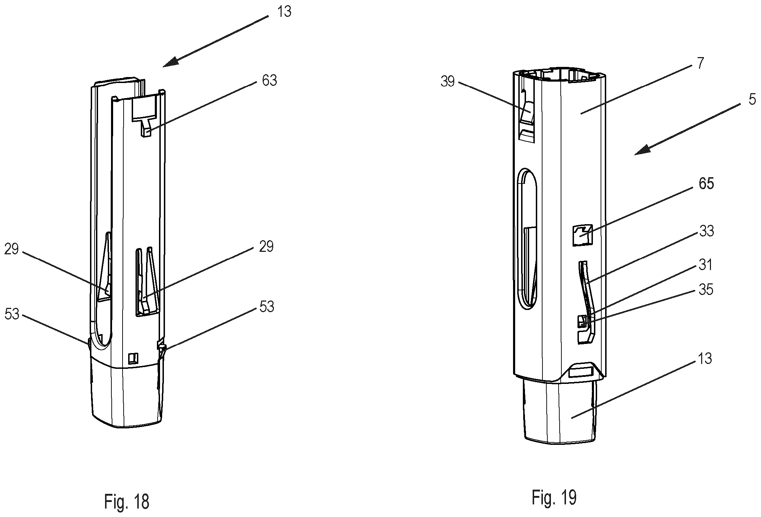

When the autoinjector 1 is removed from the skin, the needle cover 13 moves in the distal direction and returns from the retracted position to a final extended position, as shown in fig. 13 to 16. In fig. 13 to 16, the injection is completed, therefore the stopper 45 of the reservoir 23 is in its most distal position. The final extended position of the needle cover 13 is distal from the initial position of the needle cover 13, hence in the final extended position, the needle cover 13 protrudes further from the distal end of the reservoir unit housing 7 than in the initial position. The final extended position is realized by the design of the needle cover 13 and the reservoir unit housing 7 that will be explained below with reference to fig. 18 to 21. In the final extended position, the needle cover 13 is locked, hence it cannot move back in the proximal direction after the needle cover 13 has reached the final extended position. To provide the lockout function, the needle cover 13 comprises two lockout clips 53 formed opposite to each other on an outer surface of the needle cover 13 near the distal end of the needle cover 13. In the final extended position, the lockout clips 53 abut the distal end surface of the reservoir unit housing 7, as can be seen in fig. 13, so that the reservoir unit 5 cannot move in the proximal direction. Fig. 18 shows the needle cover 13 with the lockout clips 53 and the protrusions 63 to deform the plunger clips 49 in detail.

When the needle cover 13 moves in the distal direction, the pusher sleeve 17 also moves in the distal direction. As a result of the final extended position of the needle cover 13 being distal from the initial position, the pusher sleeve 17 can also move to a position that is distal from its initial position. Due to this the release arms 25 reach the reservoir clips 40 and deform the reservoir clips 40 in the radial direction as shown in fig. 16. Therefore, the projections 39 of the reservoir clips 40 are no longer in contact with the projections 37 of the drive unit housing 9. Due to this the projections 39 of the reservoir unit housing 7 can pass the projections 37 of the drive unit housing 9. By this the pusher sleeve 17 automatically releases the connection between the drive unit 3 and the reservoir unit 5 established by the connection elements 37, 39, when the needle cover 13 returns to its final extended position.

To move the needle cover 13 and the pusher sleeve 17 in the distal direction, when the autoinjector is removed from the skin, the drive unit 3 comprises a pusher sleeve spring 21 adapted to urge the pusher sleeve 17 and the needle cover 13 in the distal direction. The pusher sleeve spring 21 is arranged between a proximal end surface of the pusher sleeve 17 and a proximal inner end of the drive unit housing 9.

The autoinjector 1 further comprises two emergency eject buttons 59 shown for example in fig. 15 and 16. With the emergency eject buttons 59 the connection between the reservoir unit 5 and the drive unit 3 established by the first connection element 37 and the second connection element 39 can alternatively be released at any time by pressing on the emergency eject buttons 59. The emergency eject buttons 59 also deform the reservoir clips 40 in the radial direction, so that the projections 39 of the reservoir unit housing 7 can pass the projections 37 of the drive unit housing 9 and the reservoir unit 5 can be moved in the distal direction out of the drive unit housing 9. This is necessary for example when the injection fails to complete or if a wrong reservoir unit 5 is inserted into the drive unit 3, for example a reservoir unit 5 holding a reservoir 23 with a wrong or expired medicament.

Fig. 17 shows a cross-section of the drive unit housing 9 and the pusher sleeve 17 in detail. The two release arms 25 of the pusher sleeve 17 are each guided by a bracket 61 on an inner surface of the drive unit housing 9, one of which is visible in fig. 17.

Due to the design of the pusher sleeve 17, the needle cover 13 and the reservoir unit housing 7, that is adapted to each other, it is secured that after the injection is completed, the connection between the drive unit 3 and the reservoir unit 5 is released at the same time or after the needle cover 13 is locked in the final extended position. This is specifically enabled by channels 33 of the reservoir unit housing 7 allowing the needle cover 13 to move to the final extended position which also allows the pusher sleeve 17 to further move in the distal direction to a position distal from its initial position in combination with the length of the contact arms 27 and the release arms 25 of the pusher sleeve 17.

Fig. 18 to 21 give an overview of the design of the needle cover 13 and the reservoir unit housing 7 as well as the movement of the needle cover 13 within the reservoir unit housing 7 during the injection process.

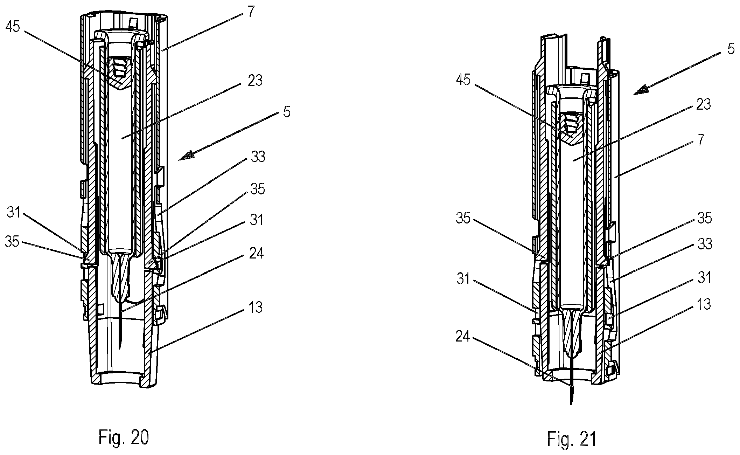

The needle cover 13 comprises two needle cover clips 29 positioned on the outer surface of the needle cover 13 opposite to each other, as shown in fig. 18. The needle cover clips 29 are elastically deformable in the radial direction and each comprise a ramped proximal surface. As shown in fig. 18, the needle cover clips 29 are positioned further away from the distal end of the needle cover 13 than the lockout clips 53. The reservoir unit housing 7 comprises two recesses 31 and two channels 33 opposite to each other, one of which is visible in fig. 19. The recesses 31 are located near the distal end of the channels 33. In the initial position of the needle cover 13 as shown in fig. 20, the needle cover clips 29 are received in the recesses 31. When the needle cover 13 is pushed in the proximal direction to the retracted position by pressing the autoinjector 1 against the skin, the needle cover clips 29 are elastically deformed due to the ramped proximal surface of the needle cover clips 29. Therefore, the needle cover clips 29 move inside the reservoir unit housing 7 by sliding on the inner surface of the reservoir unit housing 7. When the needle cover clips 29 reach a proximal end of the channels 33, the needle cover clips 29 return to their undeformed state and are received in the channels 33. When the needle cover 13 now moves in the distal direction when the autoinjector 1 is removed from the skin, the needle cover clips 29 are guided in the channels 33 to the distal end of the channels 33. When the needle cover clips 29 reach the distal end of the channels 33, the needle cover 13 is in the final extended position. The final extended position is more distal than the initial position because the distal end of the channels 33 is located more distal than the recesses 31.

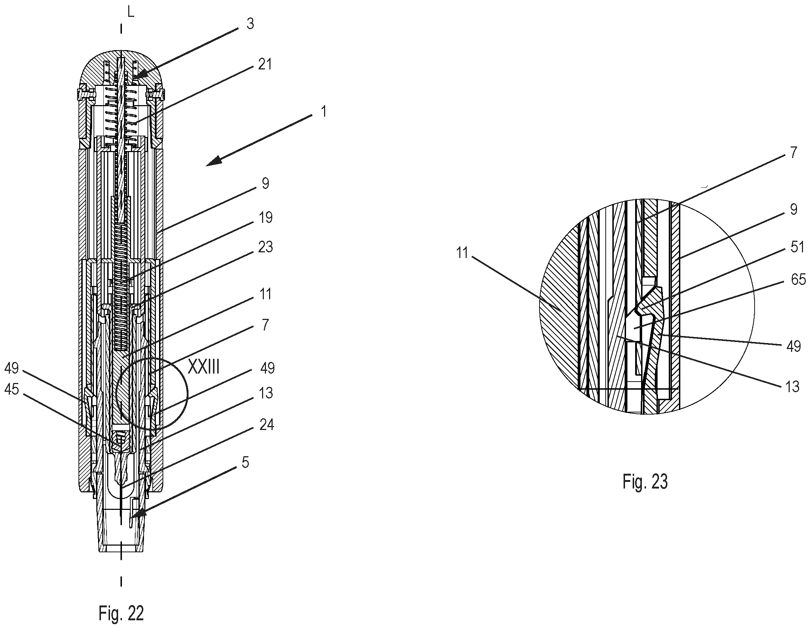

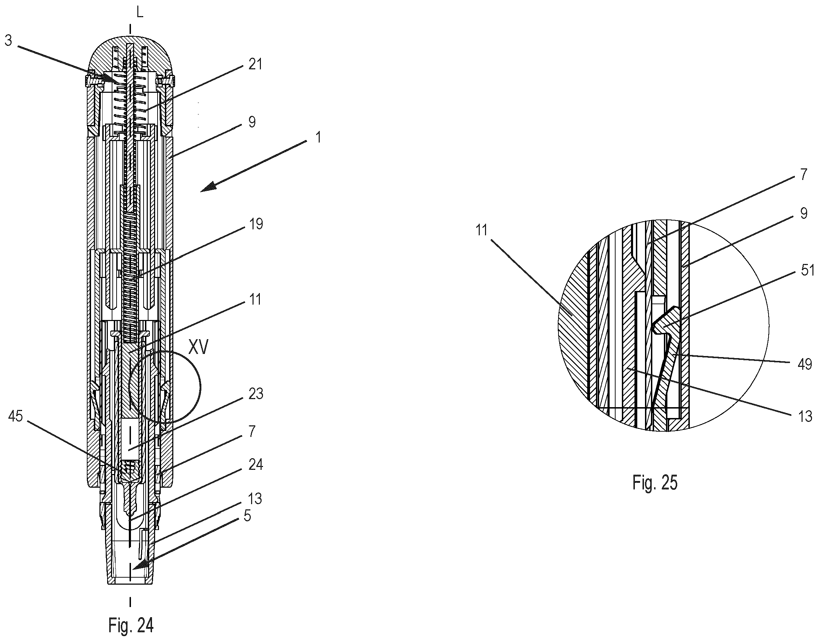

After the connection between the reservoir unit 5 and the drive unit 3 is released automatically by the pusher sleeve 17 or by pressing the emergency eject buttons 59, the reservoir unit 5 is separable from the drive unit 3 by a linear movement along the longitudinal axis L. Fig. 22 and 23 show the autoinjector 1 after the injection with the needle cover 13 locked in the final extended position and after the connection between the reservoir unit 5 and the drive unit 3 is released. In this state, the plunger clips 49 still hold the reservoir unit 5 inside the drive unit housing 9, so that the reservoir unit 5 does not fall out of the drive unit 3. To achieve this, the protrusions 51 of the plunger clips 49 are received in holding recesses 65 formed opposite to each other in the drive unit housing 7. One of the holding recesses 65 is also shown in fig. 19. Only when the user manually pulls the reservoir unit 5 out of the drive unit housing 9 in the distal direction, the plunger clips 49 are elastically deformed in the radial direction so that the protrusions 51 move out of the holding recesses 65 as shown in fig. 24 and 25. This allows the user to pull out the reservoir unit 5 along the longitudinal axis L and to separate the reservoir unit 5 from the drive unit 3.

The previous description of the disclosed embodiments is provided to enable a person skilled in the art to make or use the disclosed embodiments. Various modifications to these embodiments will be readily apparent to those skilled in the art, and the principles defined herein may be applied to other embodiments without departing from the scope of the disclosure. Thus, the present disclosure is not intended to be limited to the embodiments shown herein but is to be accorded the widest scope possible consistent with the principles and novel features as previously shown and described.

### LIST OF DESIGNATIONS

- 1: drug delivery device
- 3: drive unit
- 5: reservoir unit
- 7: reservoir unit housing
- 9: drive unit housing
- 11: plunger
- 13: needle cover
- 15: cap
- 16: needle shield
- 17: pusher sleeve
- 19: mechanical drive
- 21: pusher sleeve spring
- 23: reservoir
- 24: needle
- 25: release arm
- 27: contact arm
- 29: needle cover clip
- 31: recess
- 33: channel
- 37: first connection element
- 38: proximal abutment surface
- 39: second connection element
- 40: Reservoir clip
- 41: distal abutment surface
- 43: inner rod section plunger
- 45: stopper
- 47: plunger arm
- 49: plunger clip
- 51: projection
- 53: lockout clip
- 55: drive unit cap
- 57: mechanical drive rod
- 59: emergency eject button
- 61: bracket
- 63: protrusion
- 65: holding recess
- L: longitudinal axis

## Claims

1. A release mechanism for a drug delivery device (1) for dispensing a liquid drug from a reservoir (23) through a needle (24), the release mechanism comprising a drive unit (3) and a reservoir unit (5) adapted to hold the reservoir (23) and releasably connectable to the drive unit (3),
the drive unit (3) comprising a drive unit housing (9) defining a longitudinal axis (L), a plunger (11) movable inside the drive unit housing (9) in a dispensing direction to dispense the liquid drug and a mechanical drive (19), preferably a compression spring, arranged inside the drive unit housing (9) for moving, when loaded, the plunger (11) in the dispensing direction,
the reservoir unit (5) comprising a reservoir unit housing (7) and a needle cover (13) movable inside the reservoir unit housing (7) between an initial position, in which the needle cover (23) covers the needle (24), and a retracted position, in which the needle (24) protrudes from a distal end of the needle cover (13),
wherein the reservoir unit (5) is insertable into the drive unit housing (9) from a distal end of the drive unit housing (9), wherein the drive unit housing (9) comprises a first connection element (37) and the reservoir unit housing (7) comprises a second connection element (39) adapted to establish a connection between the drive unit (3) and the reservoir unit (5) upon contact of the first connection element (37) and the second connection element (39), during insertion of the reservoir unit (5) into the drive unit housing (9),
**characterized in that** the drive unit (3) of the release mechanism comprises a pusher sleeve (17) movable inside the drive unit housing (9) and adapted to move in a proximal direction when the needle cover (13) is moved from the initial position to the retracted position, and to move in a distal direction while releasing the connection between the drive unit (3) and the reservoir unit (5), when the needle cover (13) returns towards a final extended position.

2. Release mechanism according to claim 1, wherein the reservoir unit (5) is insertable into the drive unit housing (9) by a linear movement along the longitudinal axis (L) and/or separable from the drive unit (3) by a linear movement along the longitudinal axis (L).

3. Release mechanism according to claim 1 or 2, wherein the pusher sleeve (17) comprises a release arm (25) adapted to elastically deform the first connection element (37) or the second connection element (39) to release the connection between the reservoir unit (5) and the drive unit (3).

4. Release mechanism according to claim 3, wherein the pusher sleeve (17) further comprises a contact arm (27) with a distal end surface adapted to contact a proximal end surface of the needle cover (13), wherein preferably the pusher sleeve (17) comprises two release arms (25) and two contact arms (27) and the contact arms (27) are located radially inside the release arms (25) and/or offset to the release arms (25) in a circumferential direction.

5. Release mechanism according to one of the preceding claims further comprising a pusher sleeve spring (21) adapted to urge the pusher sleeve (17) and the needle cover (13) in the distal direction, wherein the pusher sleeve spring (21) is arranged between a proximal end surface of the pusher sleeve (17) and a proximal inner end of the drive unit housing (9).

6. Release mechanism according to one of the preceding claims, wherein the needle cover (13) comprises a needle cover clip (29) elastically deformable in the radial direction and the reservoir unit housing (9) comprises a recess (31) and a channel (33), wherein the recess (31) is adapted to receive the needle cover clip (29) in the initial position of the needle cover (13) and the channel (33) is adapted to receive the needle cover clip (29) in the retracted position of the needle cover (13) and to guide the needle cover (13) to the final extended position, wherein the final extended position is distal from the initial position.

7. Release mechanism according to claim 6, wherein the needle cover (13) comprises a lockout clip (53) positioned closer to the distal end of the needle cover (13) than the needle cover clip (29) and adapted to lock the needle cover (13) in the final extended position by abutting the distal end surface of the reservoir unit housing (7).

8. Release mechanism according to claim 7, wherein the pusher sleeve (17) is adapted to release the connection between the drive unit (3) and the reservoir unit (5) at the same time or after the needle cover (13) is locked in the final extended position.

9. Release mechanism according to one of the preceding claims, wherein the first connection element (37) comprises a projection on an inner surface of the drive unit housing (9) and the second connection element (39) comprises a projection on an outer surface of the reservoir unit housing (7), preferably at a proximal end of the reservoir unit housing (7).

10. Release mechanism according to claim 9, wherein the first connection element (37) comprises a proximal abutment surface (38) facing in the proximal direction and the second connection element (39) comprises a distal abutment surface (41) facing in the distal direction, wherein after the reservoir unit (5) is inserted into the drive unit housing (9), the first connection element (37) is positioned distal of the second connection element (39), so that the abutment surfaces (38, 41) contact each other.

11. Release mechanism according to one of the preceding claims, wherein the second connection element (39) is arranged on a free end of an elastically deformable reservoir clip (40) at a proximal end of the reservoir unit housing (7), wherein in particular the free end of the reservoir clip (40) is the distal end of the reservoir clip (35).

12. Release mechanism according to claim 11, wherein the reservoir clip (40) is adapted to be deformed in the radial direction by the first connection element (37).

13. Release mechanism according to one of the preceding claims, wherein the connection between the drive unit (3) and the reservoir unit (5) is alternatively releasable by an emergency eject button (59), wherein the second connection element (39) is deformable by the emergency eject button (59).

14. Release mechanism according to one of the preceding claims, wherein the drive unit housing (9) comprises a bracket (61) on an inner surface of the drive unit housing (9) for guiding the pusher sleeve (17).

15. A drug delivery device (1) comprising a release mechanism according to one of the preceding claims, wherein in particular the drive unit housing (9), the reservoir unit housing (7), the plunger (11), the needle cover (13) and/or the pusher sleeve (17) are injection-molded thermoplastic plastic parts and/or wherein in particular the drug delivery device (1) is a semi-reusable autoinjector with a reusable drive unit (3) and a disposable reservoir unit (5).
